# EUROPÄISCHE PATENTSCHRIFT

(11) **EP 3 166 620 B1**
(45) Veröffentlichungstag und Bekanntmachung des Hinweises auf die Patenterteilung: **23.04.2025**
(21) Anmeldenummer: 15739541.9
(22) Anmeldetag: 10.07.2015
(51) Int. Cl.: A61K 36/61, A61P 25/34, A61K 36/185, A61K 36/81, A61P 39/00, A61K 35/02

(54) **HOMÖOPATHISCHES MITTEL ZUR RAUCHERENTWÖHNUNG**
HOMÖOPATHIC REMEDY FOR SMOKING CESSATION
MEDICAMENT HOMEOPATHIQUE POUR SEVRAGE TABAGIQUE

(30) Priorität: 11.07.2014 DE 202014103194 U
(43) Veröffentlichungstag der Anmeldung: 17.05.2017
(73) Patentinhaber: Gross, Marianna, 66557 IIlingen (DE)
(72) Erfinder: Gross, Marianna, 66557 IIlingen (DE)
(74) Vertreter: Hoffmann Eitle
(86) Internationale Anmeldenummer: PCT/EP2015/065847
(87) Internationale Veröffentlichungsnummer: WO 2016/005563

(56) Entgegenhaltungen:
- WO-A1-89/09049
- DE-U1- 202008 007 923
- RU-C1- 2 143 274
- US-A1- 2006 062 835
- US-A1- 2009 142 426
- PETRA WOOD: "The Trituration of Tabacum Cubensis", 14 May 2011 (2011-05-14), pages 1, 11, Retrieved from the Internet <URL:https://www.welshschoolofhomeopathy.org.uk/provings/cuban_cigar_tobacco.pdf> [retrieved on 20180122]
- BOERIKE: "Homöopathische Mittel und ihre Wirkungen", 1 May 1986, VERLAG GRUNDLAGEN UND PRAXIS, LEER, Leer, ISBN: 3921229049, pages: 544 - 545, XP002744250

## Beschreibung

### Technisches Gebiet

Die Erfindung betrifft ein homöopathisches Mittel zur Verwendung als Arzneimittel zur Raucherentwöhnungstherapie, das Zigaretten in homöopathischer Verdünnung enthält.

### Hintergrund

Es ist bekannt, dass Rauchen schnell zu Suchterscheinungen führt. Viele Raucher, die das Rauchen aufgeben möchten, sind daher mit Entzugsproblemen konfrontiert, welche dazu führen, dass der Raucher in vielen Fällen rückfällig wird. Es sind Nikotinpflaster auf dem Markt, die eine Nikotinaufnahme über die Haut ermöglichen. Diese lösen jedoch weder das Problem der Nikotinabhängigkeit noch bieten sie einen Ersatz für den Vorgang des Rauchens, so dass auch bei Verwendung von Nikotinpflastern häufig eine dauerhafte Raucherentwöhnung scheitert.

Daneben sind auch homöopathische Mittel zur Raucherentwöhnung bekannt. DE 20 2008 007 923 U1 beschreibt ein Suppositorium, das zumindest Nicotiana tabacum enthält. Aus der US 2006/062835 A1 geht ein Verfahren zur Nikotinentwöhnung hervor, bei dem in einer ersten Phase ein Druckpad auf das Ohr des Patienten aufgebracht wird, dann der Patient in einer zweiten Phasen oral ein Entzugsmittel einnimmt, in einer dritten Phase, die sich mit der ersten Phase überlappt, eine streßreduzierende Substanz oral zu sich nimmt und in einer vierten Phase diese Behandlungsschritte fortgesetzt werden. Die US 2005/100513 A1 beschreibt ein Verfahren zur Nikotinentwöhnung, bei der gleichzeitig zwei homöopathische Substanzen verabreicht werden, wobei die erste Substanz der Nikotinsucht entgegenwirkt und die zweite zur Entgiftung des Körpers dient. In der RU 2 143 274 C1 wird schließlich ein homöopathisches Mittel zur Raucherentwöhnung beschrieben, das in Form von Zuckerkügelchen ("sugar granules") verabreicht wird. Ein weiteres Mittel zur Raucherentwöhnung auf Basis einer Mischung verschiedener Wirkstoffe, die in homöopathischer Verdünnung vorliegen, ist aus EP 2 070 544 B1 bekannt.

### Darstellung der Erfindung

Aufgabe der Erfindung ist es, ein Mittel zur Raucherentwöhnung bereitzustellen, mit dem eine dauerhafte Raucherentwöhnung in der Mehrzahl der Fälle möglich ist. Diese Aufgabe wird erfindungsgemäß durch das Mittel nach einem der Ansprüche 1-3 gelöst. Dementsprechend betrifft die vorliegende Erfindung:
i) ein homöopathisches Mittel zur Verwendung als Arzneimittel zur Raucherentwöhnungstherapie, dadurch gekennzeichnet, dass das Mittel Zigaretten, einschließlich Papier und Filter, soweit vorhanden, in homöopathischer Verdünnung enthält, dass das Mittel Zigaretten in den homöopathischen Verdünnungen D6, D8 oder D12 enthält und dass das Mittel Zigaretten der Marke in homöopathischer Verdünnung enthält, die der jeweilige Patient vor Beginn der Raucherentwöhnungstherapie geraucht hat;
ii) ein homöopathisches Mittel zur Verwendung als Arzneimittel zur Raucherentwöhnungstherapie gemäß i), dadurch gekennzeichnet, dass das homöopathische Mittel in Form von Tropfen, Globuli, einer Zuckerlösung in flüssiger oder fester Form, Kaugummis, Cremes, Salben, Gelen, Hautrollern, Suppositorien oder einer wässrigen Lösung vorliegt, wobei die wässrige Lösung zur Injektion, zum Inhalieren, zum Sprühen oder zum Verdampfen ausgebildet ist; und
iii) ein homöopathisches Mittel zur Verwendung als Arzneimittel zur Raucherentwöhnungstherapie gemäß ii), dadurch gekennzeichnet, dass die wässrige Lösung eine isotonische NaCl-Lösung ist.

Das Mittel enthält Zigaretten, einschließlich Tabakzigaretten und Haschischzigaretten, in homöopathischer Verdünnung, wobei die Zigaretten als ganzes eingesetzt werden, d.h. einschließlich Papier und Filter soweit vorhanden, und nach den Regeln des Homöopathischen Arzneibuchs (HAB) zu den gewünschten homöopathischen Verdünnungen verarbeitet werden.

Jede Zigarettenmarke enthält unterschiedliche Tabaksorten, Mischungen von Tabaksorten und Zusatzstoffe, wie z.B. Suchtverstärker, Duftstoffe, Konservierungsmittel, etc. Für eine personalisierte Therapie zur Raucherentwöhnung wird daher eine bestimmte Zigarettenmarke für das erfindungsgemäße Mittel zur Raucherentwöhnung verwendet.

Die genannten Potenzen entsprechen den allgemein üblichen und bekannten Potenzen homöopathischer Zubereitungen und werden entsprechend den Vorschriften des Homöopathischen Arzneibuchs hergestellt. So kennzeichnet der Buchstabe in Kombination mit der jeweiligen Potenzierungsstufe den Verdünnungsgrad in den erfindungsgemäßen Mitteln zur Raucherentwöhnung.

Die Herstellung des erfindungsgemäßen Mittels zur Raucherentwöhnung erfolgt nach den Regeln und Vorschriften des jeweils geltenden Homöopathischen Arzneibuchs. Danach wird aus den jeweiligen Zigaretten eine Urtinktur hergestellt und anschließend homöopathisch potenziert.

Beispielsweise kann die D6-Verdünnung einer wässrigen Lösung der Zigaretten einer bestimmten Marke als solcher gemäß HAB wie folgt hergestellt werden.

### Urtinktur:

Nach den im Abschnitt Tinkturen der Monographie Extrakte im europäischen Arzneibuch (Ph.Eur.) beschriebenen Verfahren der Mazeration oder Perkolation wird aus 1 Teil frischer oder getrockneter Zigaretten einer bestimmten Marke und 10 Teilen Ethanol geeigneter Konzentration eine Urtinktur hergestellt.

### Potenzierung:

Die Urtinktur entspricht der 1. Dezimalverdünnung D1. Die 2. Dezimalverdünnung (D2) wird aus 1 Teil der Urtinktur und 9 Teilen Wasser für Injektionszwecke hergestellt. Entsprechend wird bei den folgenden Verdünnungen verfahren, um zur 3., 4., 5. und abschließend zur 6. Dezimalverdünnung D6 zu gelangen.

Die so erhaltenen Verdünnungen können dann einzeln oder in Kombination zu geeigneten Darreichungsformen verarbeitet werden.

Die erfindungsgemäßen Mittel zur Raucherentwöhnung können in allen gängigen Darreichungsformen für die Verabreichung homöopathischer Arzneimittel vorliegen. Geeignete Darreichungsformen sind Tropfen, Globuli, eine Zuckerlösung in flüssiger oder fester Form, Kaugummis, Cremes, Salben, Gele, Hautroller, Suppositorien oder eine wässrigen Lösung, wobei die wässrige Lösung zur Injektion, zum Inhalieren, zum Sprühen oder zum Verdampfen ausgebildet sein kann.

Die wässrige Lösung kann eine isotonische NaCl-Lösung sein. Die wässrige Lösung kann auch eine alkoholische Lösung sein.

Bevorzugt liegen die erfindungsgemäßen Mittel zur Raucherentwöhnung in Form von wässrigen Lösungen zur Injektion, Tropfen und Globuli vor. Zum Beispiel können die zuvor beschriebenen D6-Verdünnungen in eine wässrige Lösung zur Injektion überführt werden.

Besonders bevorzugt ist das erfindungsgemäße Mittel zur Raucherentwöhnung eine wässrige Lösung zur Injektion. In diesem Fall ist das Verdünnungsmittel Wasser und es handelt sich um wässrige Lösungen der Wirkstoffe. Mit einer einzigen Injektion von 2 ml dieses Mittels ins Ohrläppchen ist es bei einer hohen Erfolgsquote möglich, eine sofortige Raucherentwöhnung zu erreichen. Überraschenderweise treten auch während der ersten 24 Stunden in der Regel keine Entzugserscheinungen auf und es wird in der Mehrzahl der Fälle eine langfristige Raucherentwöhnung erreicht. In seltenen Fällen ist es erforderlich, eine zweite Injektion vorzunehmen, um eine vollständige Raucherentwöhnung zu erreichen.

Das Mittel zur Raucherentwöhnung enthält Zigaretten der Marke in homöopathischer Verdünnung, die der jeweilige Patient vor Beginn der Raucherentwöhnungstherapie geraucht hat.

## Patentansprüche

1. Homöopathisches Mittel zur Verwendung als Arzneimittel zur Raucherentwöhnungstherapie, **dadurch gekennzeichnet, dass** das Mittel Zigaretten, einschließlich Papier und Filter, soweit vorhanden, in homöopathischer Verdünnung enthält, dass das Mittel Zigaretten in den homöopathischen Verdünnungen D6, D8 oder D12 enthält und dass das Mittel Zigaretten der Marke in homöopathischer Verdünnung enthält, die der jeweilige Patient vor Beginn der Raucherentwöhnungstherapie geraucht hat.

2. Homöopathisches Mittel zur Verwendung als Arzneimittel zur Raucherentwöhnungstherapie gemäß Anspruch 1, **dadurch gekennzeichnet, dass** das homöopathische Mittel in Form von Tropfen, Globuli, einer Zuckerlösung in flüssiger oder fester Form, Kaugummis, Cremes, Salben, Gelen, Hautrollern, Suppositorien oder einer wässrigen Lösung vorliegt, wobei die wässrige Lösung zur Injektion, zum Inhalieren, zum Sprühen oder zum Verdampfen ausgebildet ist.

3. Homöopathisches Mittel zur Verwendung als Arzneimittel zur Raucherentwöhnungstherapie gemäß Anspruch 2, **dadurch gekennzeichnet, dass** die wässrige Lösung eine isotonische NaCl-Lösung ist.

## Claims

1. Homeopathic agent for use as a medicament for smoking cessation therapy, **characterised in that** the agent contains cigarettes, including paper and filters, if present, in homeopathic dilution, that the agent contains cigarettes in the homeopathic dilutions D6, D8 or D12 and that the agent contains, in homeopathic dilution, cigarettes of the brand which the respective patient smoked prior to the start of the smoking cessation therapy.

2. Homeopathic agent for use as a medicament for smoking cessation therapy according to claim 1, **characterised in that** the homeopathic agent is in the form of drops, globules, a sugar solution in liquid or solid form, chewing gum, creams, ointments, gels, skin rollers, suppositories or an aqueous solution, the aqueous solution being configured for injection, inhalation, spraying or vaporisation.

3. Homeopathic agent for use as a medicament for smoking cessation therapy according to claim 2, **characterised in that** the aqueous solution is an isotonic NaCl solution.

## Revendications

1. Agent homéopathique à utiliser comme médicament pour le sevrage tabagique, **caractérisé en ce que** l'agent contient des cigarettes, y compris du papier et des filtres, le cas échéant, en dilution homéopathique, que l'agent contient des cigarettes dans les dilutions homéopathiques D6, D8 ou D12 et que l'agent contient des cigarettes de la marque en dilution homéopathique que le patient concerné a fumées avant le début du sevrage tabagique.

2. Agent homéopathique à utiliser comme médicament pour le sevrage tabagique selon la revendication 1, **caractérisé en ce que** l'agent homéopathique se présente sous forme de gouttelettes, de granules, d'une solution de sucre sous forme liquide ou solide, de chewing-gums, de crèmes, de pommades, de gels, de rouleaux cutanés, de suppositoires ou d'une solution aqueuse, dans lequel la solution aqueuse est réalisée pour l'injection, pour l'inhalation, pour la pulvérisation ou pour la vaporisation.

3. Agent homéopathique à utiliser comme médicament pour le sevrage tabagique selon la revendication 2, **caractérisé en ce que** la solution aqueuse est une solution de NaCl isotonique.
